# EUROPEAN PATENT SPECIFICATION

(11) **EP 2 296 717 B1**
(45) Date of publication and mention of the grant of the patent: **05.09.2012**
(21) Application number: 09749768.9
(22) Date of filing: 15.05.2009
(51) Int. Cl.: A61L 15/46, A61F 13/00

(54) **FISTULA PAD**
FISTELPOLSTER
TAMPON POUR FISTULE

(30) Priority: 19.05.2008 IT BO20080303
(43) Date of publication of application: 23.03.2011
(73) Proprietor: Emodial S.r.l., 44100 Cassana (Ferrara) (IT)
(72) Inventor: PECORARI, Gianni, 41037 Mirandola (Modena) (IT)
(74) Representative: Dall'Olio, Giancarlo
(86) International application number: PCT/EP2009/055921
(87) International publication number: WO 2009/141278

(56) References cited:
- WO-A2-98/41095
- US-A1- 2004 259 445
- US-A1- 2007 179 522

## Description

### TECHNICAL FIELD

The invention relates to a new fistula pad comprising at least a substance selected from the group of silver, aluminium or derivatives thereof.

It is known that patients suffering from kidney failure need haemodialysis, which is a treatment allowing blood purification by means of a suitable equipment: the kidney machine.

### BACKGROUND ART

The haemodialysis, which requires extracorporeal circulation of the patient's blood, needs a vascular access, that is to say a point where two needles are positioned; one needle withdraws the blood to be purified, the other one returns the filtered blood to the body.

Currently, three types of accesses are employed: arteriovenous fistula, artificial grafts (prosthesis) and catheters in the jugular, subclavian or femoral vein. Fistula is the most employed vascular access. To create a fistula, the patient is subjected to a surgical intervention, wherein a permanent connection between a vein and an artery is provided. Since the arterial pressure is much higher than the venous one, after the intervention the arterial blood causes the enlargement of the vein. The fistula is therefore aimed at enlarging the vein volume. Such vein, which is defined arterialized, is employed as access for dialysis since it allows the passage of high blood flows favouring the dialysis process.

The haemodialysis is a long treatment which is repeated in the time; each session lasts between three and four hours and it is performed two/three times per week. As a consequence, the arterialized vein in which the fistula was created is continually subjected to traumas due to the insertion of the needles, defined "fistula needles", which are provided with diameters in the range of 1,6 mm.

Since high blood flows flow in the arterialized vein and since the patients subjected to dialysis are given heparin or other anticoagulant agents to prevent the blood coagulation in the dialyzer, it is important that the wounds due to the insertion of the fistula needles heal as quickly as possible. This is needed both to prevent recurrent and significant bleeding of the patient, and to minimize the probability that the health care professional comes in contact with the blood of the patient, or that the wound becomes infected.

The fistula pads currently available on the market distinguish themselves from the other blood or exudates absorbing devices since they are provided with a thicker absorbing layer as well as a greater rigidity. Such rigidity allows said fistula pads to distribute uniformly the pressure exerted on the hole made by the fistula needle, thus favouring the processes of haemostasis and vein healing. Said rigidity is due to the presence of a structural element provided with greater rigidity than the one of the absorbing layer. Utility model patent n° IT244964U of the applicant describes a multi-layer fistula pad wherein several layers of absorbing material are held together by means of both-sides adhesive impermeable films. In addition, the impermeable films function as containing barrier isolating the wound from possible outer contamination and preventing the blood coming out from the wound to come in contact with the health care professional who is applying the fistula pad.

Despite the usage of these specific fistula pads, the total bleeding of a patient periodically subjected to haemodialysis for a rather long period of time, is however remarkable. Moreover the holes caused by the fistula needles heal in a relatively long time with respect to the holes caused by other needle types, thus increasing the probability of microbiological contamination of the arteriovenous fistula, along with the possible risk of infections onset in the patients.

Therefore there exists the need for a better fistula pad which allows to further minimize the side effects of the aforementioned haemodialysis.

### DISCLOSURE OF INVENTION

Therefore an object of the present invention is to provide a improved fistula pad which reduces the probability that the wound becomes infected and that allows the hole made by the needle to heal in a shorter time with respect to what can be achieved by means of the known pad.

Another object of the present invention is to limit the blood coming out from the hole made by the fistula needle.

The aforementioned objects are achieved by means of a fistula pad comprising:
- at least a superficial layer, which is intended to be placed on the hole made by the needle, and which is permeable to blood and body exudates;
- at least an absorbing layer;
- at least an outer layer, which is more rigid than a single absorbing layer, and wherein said absorbing layer is arranged between said superficial layer and said outer layer, and at least one of said superficial layer and absorbing layer comprises at least a substance selected from the group of silver, aluminium and derivatives thereof selected from the group consisting of aluminium sulphate, anhydrous and in its various hydration forms; aluminium potassium dodecahydrate; metal silver ionic silver; silver nitrate, proteinic silver, vitellinate silver, silver alginate and silver sulfadiazine.

The presence of silver, aluminium or derivatives thereof favours the haemostasis thus reducing the healing time of the hole made by the fistula needle and by the bleeding. Moreover it gives the fistula pad anti-bacterial features, thus reducing the probability of wound infection.

Said superficial layer is preferably anti-adherent; it is free of fibrous projecting particles and the outer layer is impermeable to blood and body exudates.

The absorbing layer can be made up of absorbing hydrophilic cotton gauze or non-woven fabric (NWF) or expandable dressings made up of a single spongy material as for example synthetic felt or polypropylene staple fibres, pharmaceutical grade viscose. An absorbing layer in viscose or viscose-polypropilene having a thickness between 0,1 and 15 mm or greater is preferred according to the clinical situation. A thickness between 1 and 4 mm with an absorbing power of 500-1500% in a second, more preferably 700-1500% in a second, is more preferred.

### BRIEF DESCRIPTION OF THE DRAWINGS

Fig. 1 is a perspective view of an embodiment of the invention;
Fig. 2 is a perspective view of another embodiment of the invention;
Fig. 3 is a perspective view of another embodiment of the invention;
Fig. 4 is a perspective view of another embodiment of the invention.

### BEST MODE FOR CARRYING OUT THE INVENTION

Throughout the various views 1-4, identical elements are indicated with the same reference numbers of the following description of the various embodiments of the invention and suggest similar features, unless it is indicated otherwise.

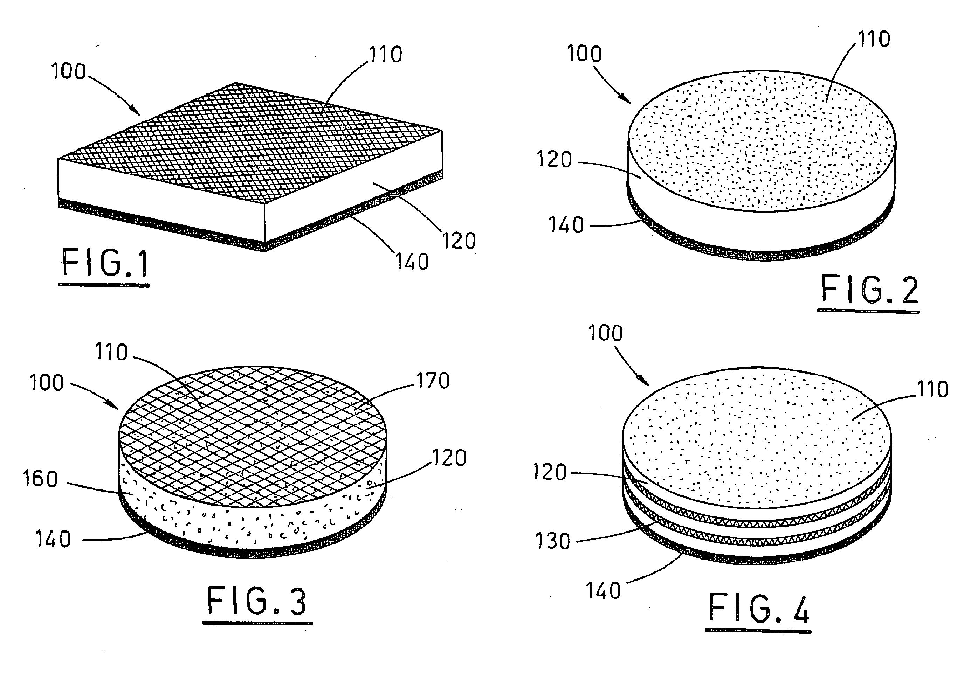

In at least an embodiment of the fistula pad 100, the superficial layer 110 is an anti-adherent fabric, an anti-adherent mesh, an anti-adherent non-woven fabric (NWF), whose fibres are in silver or aluminium or a perforated thin layer of silver or aluminium. In figure 1, for example, there is shown a fistula pad 100, wherein the superficial layer 110 is an anti-adherent fabric, whose fibres can be in aluminium or silver.

In at least an embodiment the superficial layer 110 is an anti-adherent fabric, an anti-adherent mesh and an anti-adherent non-woven fabric (NWF) whose fibres are covered by silver or aluminium, and wherein the aluminium or silver fibres are arranged on the surface of the superficial layer intended to be put in contact with the hole made by the fistula needle. In such case a support, which can be anti-adherent and permeable to blood and to body exudates and which can be a mesh, a fabric, a NWF, in polyethylene, polyester, viscose or other pharmaceutical grade material, is metallized with silver and/or aluminium. The metallization can be performed both by means of classic techniques and new techniques, as for example micro or nano-deposition. By means of the metallization of the support, the quantity of silver or metal employed is reduced with respect to the embodiment in which the superficial layer 110 is a fabric, a mesh, a non-woven fabric (NWF) whose fibres are in silver or aluminium. In fig. 2 it is shown an embodiment whose superficial layer 110 is a NWF, metallized with silver or aluminium.

In an alternative embodiment, there are employed silver and/or aluminium derivatives or metal particles 160 of silver and/or aluminium which can be arranged on the superficial layer 110 and/or in the absorbing layer 120. In figure 3 it is shown an embodiment wherein a plurality of metal particles 160 of silver and/or aluminium is arranged inside the absorbing layer 120.

Fistula pads comprising at least one of the following aluminium and silver derivatives, selected from the group of aluminium sulphate (anhydrous and in its various hydration forms; cas number 10043-01-03, 16828-11-8, 16828-12-9 and 7784-31-8), aluminium and potassium dodecahydrate (also known as alum) cas number 7784-24-9; metal silver; ionic silver; an inorganic silver salt, in particular silver nitrate cas number 7761-88-8; and an organic silver compound, in particular proteinic silver documents 9008-42-8 vitellinate silver, cas number 9015-51-4, silver alginate and silver sulfadiazine, are particularly preferred.

When the metal particles 160 of silver and/or aluminium or derivatives thereof are contained only in the absorbing layer 120 (as shown for example in figure 3), the superficial layer 110 of the fistula pad is provided with a structure with holes or openings 170 which allow the absorbing layer to come in contact with the skin of the patient, so that silver, aluminium or derivatives thereof can perform their haemostatic function. However, the structure of the superficial layer holds the fibres of the absorbing layer inside the pad during the removing step of the same from the skin as well. According to the absorbing material type and to the fibres length, the expert in the art is perfectly able to determine the holes and the openings shape. In these embodiments, the superficial layer can be made up of a fabric or a mesh in cotton yarn, polyester or other material employed in the production of superficial layers of absorbing medical devices and well known to the expert in the art.

The usage of silver or its derivatives is preferable with respect to the usage of aluminium or its derivatives since silver is provided with greater anti-bacterial and haemostatic power than aluminium or its derivatives. The fistula pads according to the invention, comprising silver or its derivatives are therefore preferred.

Figure 4 shows another alternative embodiment wherein a plurality of superposed absorbing layers 120 is interposed between the superficial layer 110 made up of metallized NWF and the outer layer 140. The absorbing layers 120 adhere with respect to each other by means of an adhesive film 130. Such adhesive film is at least partially permeable to blood and body exudates.

The fistula pads according to the invention can be produced in different geometric shapes with profiles, which can be square, circular as shown in fig. 1-4, oval, rectangular, triangular etc...; or they can be provided as plates which can be cut directly by the health care professional when used.

The not shown embodiments, wherein the fistula pad is applied on a patch, an elastic bandage, an auto-adherent bandage or a fistula pressing device in plastic material are particularly advantageous, since they allow to auto-compress the hole made by the needle without requiring another manual compression.

Optionally, in case of fistula pads in which silver, aluminium or derivatives thereof are arranged only on the superficial layer 110, the absorbing layer 120 can be partially soaked in an anti-bacterial agent as chlorexidine.

## Claims

1. Fistula pad comprising:
- at least a superficial layer (110), which is intended to be placed on the hole made by the needle, and which is permeable to blood and body exudates;
- at least an absorbing layer (120);
- at least an outer layer (140), which is more rigid than a single absorbing layer (120), and
wherein said absorbing layer is arranged between said superficial layer (110) and said outer layer (140), **characterized in that** at least one of said superficial layer and absorbing layer comprises at least a substance selected from the group of silver, aluminium and derivatives thereof selected from the group consisting of aluminium sulphate, anhydrous and in its various hydration forms; aluminium potassium dodecahydrate; metal silver; ionic silver; silver nitrate, proteinic silver, vitellinate silver, silver alginate and silver sulfadiazine.

2. Fistula pad according to claim 1, wherein said first superficial layer (110) is an anti-adherent fabric, an anti-adherent mesh, an anti-adherent non-woven fabric whose fibres are In silver or aluminium or a perforated thin layer of silver or aluminium.

3. Fistula pad according to claim 1, wherein said superficial layer (110) is an anti-adherent fabric, an anti-adherent mesh or an anti-adherent non-woven fabric (NWF) having fibres covered of silver or aluminium and wherein said fibres of aluminium or silver are arranged on the surface of the superficial layer (110) intended to be put in contact with the hole made by the fistula needle.

4. Fistula pad according to any one of claims 1-3, wherein said silver and/or aluminium derivatives or metal particles (160) of silver and/or aluminium are arranged on the superficial layer (110) and/or in the absorbing layer (120).

5. Fistula pad according to any one of claims 1-4, wherein at least one of said superficial layer and absorbing layer comprises at least one of the following aluminium and silver derivatives selected from the group of aluminium sulphate, anhydrous and in its various hydration forms; aluminium and potassium dodecahydrate; metal silver; ionic silver; an inorganic silver salt and an organic silver compound.

6. Fistula pad according to any one of claims 1-5, wherein at least one of said superficial layer and absorbing layer comprises at least one of the following silver derivatives selected from the group of silver nitrate, proteinic silver, vitellinate silver, silver alginate and silver sulfadiazine.

7. Fistula pad according to any one of claims 1-6, wherein at least one of said superficial layer and absorbing layer comprises silver or its derivatives.

8. Fistula pad according to any one of claims 1-7, wherein said superficial layer (110) is anti-adherent and free of projecting fibrous particles.

9. Fistula pad according to any one of claims 1-8, wherein the outer layer is impermeable to blood and body exudates.

10. Fistula pad according to any one of claims 1-9, wherein a plurality of superposed absorbing layers (120) is interposed between said superficial layer (110) and said outer layer (140), each absorbing layer (120) of the plurality of absorbing layers adhering to the other one by means of an adhesive film (130), which is at least partially permeable to blood and body exudates.

11. Fistula pad according to any one of claims 1-10, wherein said absorbing layer (120) is made up of an absorbing material selected from the group of hydrophilic cotton gauze, non-woven fabric, expandable dressings made up of a single spongy material, synthetic felts, polypropylene staple fibres and pharmaceutical grade viscose.

12. Fistula pad according to claim 11, wherein said material is viscose.

13. Fistula pad according to any one of claims 1-12, wherein said at least one absorbing layer (120) is partially soaked in chlorexidine.

14. Fistula pad according to any one of claims 1-13, having different geometric shapes with square, circular, oval, rectangular, triangular profiles or being provided in plates, which can be cut directly by the health care professional when used.

15. Fistula pad according to any one of claims 1-14, applied on a patch, an elastic bandage, an auto-adherent bandage or a fistula pressing device in plastic material.

## Patentansprüche

1. Fistelkompresse, Folgendes beinhaltend:
- zumindest eine Oberflächenschicht (110), die dazu vorgesehen ist, auf die Einstichöffnung der Nadel aufgelegt zu werden und die durchlässig für Blut und Körperexsudate ist;
- zumindest eine absorbierende Schicht (120);
- zumindest eine Außenschicht (140), die steifer ist als eine einzelne absorbierende Schicht (120), und
worin die absorbierende Schicht zwischen der Oberflächenschicht (110) und der Außenschicht (140) angeordnet ist, **dadurch gekennzeichnet, dass** zumindest eine Schicht der genannten Oberflächenschicht und der genannten absorbierenden Schicht zumindest eine Substanz beinhaltet, die ausgewählt ist aus der Gruppe bestehend aus Silber, Aluminium und deren Derivaten, ausgewählt aus der Gruppe bestehend aus Aluminiumsulfat, wasserfrei und in seinen verschiedenen hydratisierten Formen; Aluminiumkaliumsulfat-Dodecahydrat; metallisches Silber; ionisches Silber; Silbernitrat, Silbereiweiß (Argentum proteinicum), Vitellinsilber, Silberalginat und Silbersulfadiazin.

2. Fistelkompresse nach Anspruch 1, worin die erste Oberflächenschicht (110) aus einem nicht anhaftenden Gewebe, einem nicht anhaftenden Gestrick/Gewirke oder einem nicht anhaftenden nicht gewebten Flächengebilde (Vlies) besteht, dessen Fasern aus Silber oder Alummium bestehen, oder aus einer perforierten dünnen Schicht aus Silber oder Aluminium.

3. Fistelkompresse nach Anspruch 1, worin die Oberflächenschicht (110) aus einem nicht anhaftenden Gewebe, einem nicht anhaftenden Gestrick/Gewirke oder einem nicht anhaftenden nicht gewebten Flächengebilde (Vlies) besteht, dessen Fasern mit Silber oder Aluminium überzogen sind und worin die Fasern aus Aluminium oder Silber auf der Oberfläche der Oberflächenschicht (110) angeordnet sind und dazu vorgesehen sind, in Berührung mit der Einstichöffnung der Fistelnnadel aufgelegt zu werden.

4. Fistelkompresse nach einem der Ansprüche 1-3, worin die Silber-und/oder Aluminiumderivate oder Metallpartikel (160) aus Silber und/oder Aluminium auf der Oberflächenschicht (110) und/oder in der absorbierenden Schicht (120) angeordnet sind.

5. Fistelkompresse nach einem der Ansprüche 1-4, worin zumindest eine Schicht der genannten Oberflächenschicht und der genannten absorbierenden Schicht zumindest eines der folgenden Aluminium- und Silberderivate beinhaltet, das ausgewählt ist aus der Gruppe bestehend aus Aluminiumsulfat, wasserfrei und in seinen verschiedenen hydratisierten Formen; Aluminiumkaliumsulfat-Dodecahydrat; metallisches Silber; ionisches Silber; einem anorganischen Silbersalz und einer organischen Silberverbindung.

6. Fistelkompresse nach einem der Ansprüche 1-5, worin zumindest eine Schicht der genannten Oberflächenschicht und der genannten absorbierenden Schicht zumindest eines der folgenden Silberderivate beinhaltet, das ausgewählt ist aus der Gruppe bestehend aus Silbernitrat, Silbereiweiß (Argentum proteinicum), Vitellinsilber, Silberalginat und Silbersulfadiazin.

7. Fistelkompresse nach einem der Ansprüche 1-6; worin zumindest eine Schicht der genannten Oberflächenschicht und der genannten absorbierenden Schicht Silber oder eines seiner Derivate beinhaltet.

8. Fistelkompresse nach einem der Ansprüche 1-7, worin die Oberflächenschicht (110) nicht anhaftend ist und frei von hervorstehenden faserigen Partikeln ist.

9. Fistelkompresse nach einem der Ansprüche 1-8, worin die Außenschicht undurchlässig für Blut und Körperexsudate ist.

10. Fistelkompresse nach einem der Ansprüche 1-9, worin mehrere einander überlagerte absorbierende Schichten (120) zwischen der Oberflächenschicht (110) und der Außenschicht (140) angeordnet sind, wobei jede absorbierende Schicht (120) dieser Reihe von absorbierenden Schichten durch Wirkung eines Klebefilms (130) aneinander anhaften, der zumindest teilweise durchlässig für Blut und Körperexsudate ist.

11. Fistelkompresse nach einem der Ansprüche 1-10, worin die absorbierende Schicht (120) aus einem absorbierenden Material besteht, das ausgewählt ist aus der Gruppe von hydrophiler Baumwollgaze, nicht gewebten Flächengebilden (Vlies), ausdehnbaren Verbänden aus einem einzigen schwammartigen Material, synthetischen Filzen, Polypropylen-Stapelfasern und Viskose in pharmazeutischer Qualität.

12. Fistelkompresse nach Anspruch 11, worin das Material aus Viskose besteht.

13. Fistelkompresse nach einem der Ansprüche 1-12, worin die zumindest eine absorbierende Schicht (120) teilweise mit Chlorhexidin getränkt ist.

14. Fistelkompresse nach einem der Ansprüche 1-13, die verschiedene geometrische Formen mit quadratischen, kreisförmigen, ovalen, rechteckigen oder dreieckigen Profilen aufweist oder in Form von Platten bereitgestellt wird, die direkt beim Gebrauch von dem medizinischen Pflegepersonal zugeschnitten werden können.

15. Fistelkompresse nach einem der Ansprüche 1-14, die auf ein Pflaster, eine elastische Binde, einen selbsthaftenden Verband oder eine Kompressionsvorrichtung aus Kunststoff für Fisteln aufgebracht ist.

## Revendications

1. Un tampon pour fistule, comprenant:
- au moins une couche superficielle (110), qui est destinée à être placée sur le trou fait par l'aiguille, et qui est perméable au sang et aux exsudats corporels ;
- au moins une couche absorbante (120) ;
- au moins une couche extérieure (140), qui est plus rigide qu'une seule couche absorbante (120), et
dans lequel ladite couche absorbante est disposée entre ladite couche superficielle (110) et ladite couche extérieure (140), **caractérisé en ce qu'**au moins une desdites couche superficielle et couche absorbante comprend au moins une substance choisie dans le groupe comprenant argent, aluminium et dérivés de ceux-ci choisis dans le groupe constitué par sulfate d'aluminium, anhydre et dans ses différentes formes d'hydratation ; aluminium et potassium dodécahydraté, argent métallique ; argent ionique ; nitrate d'argent, argent protéinique, argent vitellinate, alginate d'argent et sulfadiazine d'argent.

2. Le tampon pour fistule selon la revendication 1, **caractérisé en ce que** ladite première couche superficielle (110) est un tissu anti-adhérent, une maille anti-adhérente, un tissu non tissé anti-adhérent dont les fibres sont en argent ou aluminium ou une fine couche perforée d'argent ou aluminium.

3. Le tampon pour fistule selon la revendication 1, **caractérisé en ce que** ladite couche superficielle (110) est un tissu anti-adhérent, une maille anti-adhérente ou un tissu non tissé (TNT) anti-adhérent ayant des fibres recouvertes d'argent ou aluminium et **caractérisé en ce que** lesdites fibres d'aluminium ou argent sont disposées sur la surface de la couche superficielle (110) destinée à être placée au contact du trou fait par l'aiguille de la fistule.

4. Le tampon pour fistule selon l'une quelconque des revendications de 1 à 3, **caractérisé en ce que** lesdits dérivés d'argent et/ou aluminium ou des particules métalliques (160) d'argent et/ou aluminium sont disposés sur la couche superficielle (110) et/ou dans la couche absorbante (120).

5. Le tampon pour fistule selon l'une quelconque des revendications de 1 à 4, **caractérisé en ce qu'**au moins une desdites couche superficielle et couche absorbante comprend au moins un des dérivés suivants d'aluminium et argent choisi dans le groupe constitué par sulfate d'aluminium, anhydre et dans ses différentes formes d'hydratation ; aluminium et potassium dodécahydraté ; argent métallique ; argent ionique ; un sel d'argent inorganique et un composé d'argent organique.

6. Le tampon pour fistule selon l'une quelconque des revendications de 1 à 5, **caractérisé en ce qu'**au moins une desdites couche superficielle et couche absorbante comprend au moins un des dérivés suivants d'argent choisi dans le groupe constitué par nitrate d'argent, argent protéinique, argent vitellinate, alginate d'argent et sulfadiazine d'argent.

7. Le tampon pour fistule selon l'une quelconque des revendications de 1 à 6, **caractérisé en ce qu'**au moins une desdites couche superficielle et couche absorbante comprend de l'argent ou ses dérivés.

8. Le tampon pour fistule selon l'une quelconque des revendications de 1 à 7, **caractérisé en ce que** ladite couche superficielle (110) est anti-adhérente et privée de particules fibreuses saillantes.

9. Le tampon pour fistule selon l'une quelconque des revendications de 1 à 8, **caractérisé en ce que** la couche extérieure est imperméable au sang et aux exsudats corporels.

10. Le tampon pour fistule selon l'une quelconque des revendications de 1 à 9, **caractérisé en ce qu'**une pluralité de couches absorbantes (120) superposées est interposée entre ladite couche superficielle (110) et ladite couche extérieure (140), chaque couche absorbante (120) de ladite pluralité de couches absorbantes adhérant à l'autre par l'intermédiaire d'un film adhésif (130) qui est au moins partiellement perméable au sang et aux exsudats corporels.

11. Le tampon pour fistule selon l'une quelconque des revendications de 1 à 10, **caractérisé en ce que** ladite couche absorbante (120) est réalisée dans un matériau absorbant choisi dans le groupe constitué par gaze en coton hydrophile, tissu non tissé, tampons expansibles réalisés dans un unique matériau spongieux, feutres synthétiques, fibres discontinues de polypropylène et viscose de grade pharmaceutique.

12. Le tampon pour fistule selon la revendication 11, **caractérisé en ce que** ledit matériau est de la viscose.

13. Le tampon pour fistule selon l'une quelconque des revendications de 1 à 12, **caractérisé en ce que** ladite au moins une couche absorbante (120) est partiellement imbibée de chlorhexidine.

14. Le tampon pour fistule selon l'une quelconque des revendications de 1 à 13, ayant différentes formes géométriques à profils carrés, circulaires, ovales, rectangulaires, triangulaires, ou étant fourni en plaques qui peuvent être directement découpées par le professionnel de la santé au moment de l'utilisation.

15. Le tampon pour fistule selon l'une quelconque des revendications de 1 à 14, appliqué sur un sparadrap, une bande élastique, une bande auto-adhérente ou un dispositif presse-fistule en matière plastique.
